# EUROPEAN PATENT APPLICATION

(11) **EP 4 821 471 A1**
(43) Date of publication of application: **14.10.2026**
(21) Application number: 25169008.7
(22) Date of filing: 08.04.2025
(51) Int. Cl.: A61F 13/15, D04H 1/425, B31F 7/00

(54) **METHOD AND APPARATUS FOR EMBOSSING SEPARATED WEB SECTIONS WITH SHORT FIBERS**

(71) Applicant: COAX Technologies S.r.l., 26010 Monte Cremasco Cremona (IT)
(72) Inventor: ZAMPOLLO, Fabio, 26013 Crema (IT)
(74) Representative: Plischke, Manfred

(57) **Abstract**

The present invention is an apparatus and a process for operating such an apparatus for embossing a series of separated fibrous web sections preferably of the air-laying type. Such structures may suitably be absorbent, and may be used for or as bed-pads or for other absorbent articles, and may comprise short, preferably cellulosic, fibers, preferably of a fiber length of less than about 1.5 mm, such as eucalyptus fibers, and particulate material, such as superabsorbent particles.

## Description

### Field of the invention

The present invention relates to an apparatus and a process for operating such an apparatus for embossing a series of separated fibrous web sections, preferably of the air-laying type. Such structures may suitably be absorbent, and may be used for or as bed-pads or for other absorbent articles, and may comprise short, preferably cellulosic, fibers, preferably of a fiber length of less than about 1.5 mm, such as eucalyptus fibers, and particulate material, such as superabsorbent polymer particles.

### Background

Fibrous sheet materials, particularly fibrous sheet materials for absorbing fluids, are manufactured for many uses, for example they are incorporated into absorbent articles such as disposable diapers, incontinent bed-pads and catamenial napkins as fluid absorption or fluid transmission and/or diffusion elements, for example, as absorbent cores that are intended to absorb and retain body fluids.

Dry laying and, more specifically, air laying processes are widely used to produce webs from dry fibers, which can in turn be used e.g., as sheet materials for absorbing fluids. Particularly, the air laying process refers to the formation of webs with a random fiber orientation. The fibrous sheet materials produced by air laying processes are soft, flexible and porous, and are particularly suitable for use as liquid absorbent structures in absorbent articles, such as disposable diapers, sanitary napkins, pantiliners, incontinent or bed-pads, and wipes. Air laying processes are well known in the art, as are improvements aiming at evenly distributing the fibers in the airlaid structure and/or enhancing processability.

In US5527171 (Niro) the deposition of air suspended fibers in homogeneous layers is described by using multiple rows of rotating impellers in a forming head.

Similarly, US6233787 (Dan-Web / Advanced Nonwoven S/A) shows an apparatus for uniformly distributing a disintegrated material on a fiber layer forming surface by a stirrer having impellers rotating a short distance above a collecting surface.

US20030070262 (Oerlikon) describes two air-lay stations placed one after the other serving for the dry production of a non-woven fiber web, the stations including a fiber feed duct, an air laying forming head, a perforated screen as collecting surface, and a suction box for supporting the deposition of the fibers on the screen. A separate fiber source is part of each station.

Further, WO200004232 (M&J) describes the production of a nonwoven web of fibers out of a fibrous material, such as cellulose fluff pulp. The fibrous material is disintegrated by a hammer mill and deposited at the outlet of a forming head on an endless collection wire, thereby forming a web of fibers. Nits are extracted from the forming head via a transport fan and a second air duct. Furthermore, the nits are defibrated and returned to the forming head.

Also, WO2004106604A1 (Oerlikon) aims at avoiding nits by employing a fiber distributor in a forming head at optimizing the rotational speed of the wings of the distributor.

Co-pending EP24188478 (unpublished, CoAx) describes the formation of separated air-laid structures, as may be absorbent structures, such as for absorbent articles.

However, applicant identified an outstanding need for an easy to execute bonding of the fibrous structure without excessive loss of fibers and particles, if present.

Thus, the present invention provides an equipment and a corresponding manufacturing process for embossing fibrous web sections.

### Summary

In a first aspect, the present invention is an embossing unit for embossing fibrous web sections, which comprises
- a vacuum anvil drum with a web support surface, the web support surface comprising a multiplicity of apertures arranged in an aperture pattern,
   and
- a counteracting embossing drum comprising on its outer surface a multiplicity of raised embossing protrusions, each exhibiting an outwardly oriented embossing surface on the enveloping cylinder surface circumscribing the raised embossing protrusions, whereby the raised embossing protrusion surfaces forming an embossing protrusions surface pattern.

Further,
- the apertures exhibit an equivalent diameter of less than about 10 mm,
- the raised embossing protrusions exhibit an outwardly oriented embossing surface exhibiting a circular equivalent area of
   o less than about 20 mm,
   o and more than about 0.15 mm;
- the raised embossing protrusions surfaces exhibit a center point to neighbouring center point distance of less than about 5 mm;
- the apertures and the raised protrusions are arranged such that in a juxtaposition of a flat projection of the aperture pattern and the embossing protrusion surfaces pattern the apertures and the protrusion surfaces do not overlap. Preferably, the anvil and the embossing roll of the embossing unit are adapted to be operated at synchronized speed and circular position.

The aperture pattern and the embossing protrusion surfaces pattern may comprise separation regions, which are free of apertures and embossing protrusions.

Further, the raised protrusion surfaces may exhibit a shape selected from the group consisting of circle, oval, polygon, star, rhomb, triangle, or crescent, and are preferably a circle or oval.

In another aspect, the present invention is a process for embossing a series of separated fibrous web sections, preferably liquid absorbent fibrous web sections, whereby the process comprises the steps of
- providing manufacturing units of
   o a web pieces supply unit, preferably a foraminous belt system for providing a series of essentially unbonded fibrous web sections, preferably air-laid web pieces, the web sections comprising
      □ short fibers, preferably cellulosic fibers, preferably exhibiting an average fiber length of less than about 1.5 mm, more preferably of less than about 1.0 mm,
      □ and optionally particulate material, preferably superabsorbent material,
   o an embossing unit for embossing the series of separated fibrous web sections, comprising
      - a vacuum anvil drum with a web support surface, the web support surface comprising a multiplicity / plurality of apertures, arranged in an aperture pattern,
      - and
      - a counteracting embossing drum comprising on its outer surface a multiplicity of raised embossing protrusions exhibiting an outwardly oriented embossing surface on the enveloping cylinder surface circumscribing the raised embossing protrusions, whereby the raised embossing protrusions form an embossing protrusions pattern;
   o a first transfer unit for transferring the series of separated web sections to the embossing unit, preferably a vacuum transfer drum;
   o a first envelope web supply for providing a continuous envelope web, preferably a fluid pervious web and preferably a first envelope web glue applicator for applying glue to the first envelope web;
   ∘ optionally a further envelope web supply for providing a continuous envelope web, preferably a fluid impervious web and a further envelope web glue applicator for applying glue to the further envelope web;
   o a transfer and combining unit for receiving the embossed web sections from the embossing unit and for combing these with the envelope web(s);
   o a web removal unit, preferably a foraminous belt system,
      whereby the manufacturing units are arranged to define an upstream to downstream path
   o from the web pieces supply,
   o to the transfer unit;
   o to theembossing unit;
   o to the further transfer and combining unit;
   o to the web removal unit;
- delivering the series of fibrous web sections on the supply unit towards the first transfer unit/ drum;
- transferring the fibrous web sections by the first transfer unit to the embossing unit;
- embossing the fibrous web sections by the embossing unit in regions of the protrusion pattern but not in regions of the aperture pattern,
   o thereby operating the embossing unit synchronized in speed and rotative position to the series of fibrous web sections;
- optionally applying glue to the first envelope web, preferably in a pattern corresponding in length and position to the series of web sections
- transferring the embossed web sections and the carrier web to the transfer and combing unit;
- transferring the carrier web with the web pieces towards the removal unit and to further downstream processing units.

The embossing unit is preferably operated so as to increase the internal strength of the web sections by creating hydrogen bonding between fibers of the fibrous web sections.

### Brief description of the Figures

Fig. 1 A to G show an equipment for operating the present invention of embossing a series of separated fibrous structures.
Fig. 2 A to I show particular elements of equipment for operating the present invention of forming a series of separated fibrous structures.

The figures are schematic only, and not to scale. Same numerals refer to same or equivalent features or elements, single (') or multiple (", ‴, ...) apostrophes indicate duplicate features, such a left and right or front and back, etc. Omitting features in one figure does not imply that these cannot be combined with features of other figures.

### Detailed description

In a first aspect, the present invention is an apparatus for embossing a series of separate fibrous web sections, such as may be air-laid webs formed by an air-laying process, and which may comprise particulate material. In a second aspect, the present invention is a process for embossing series of separate fibrous web sections, such as may formed by an air-laying process, and which may comprise particulate material.

Wood pulp fibers are a preferred starting material as may be formed by a variety of pulping processes, such as kraft pulp, sulfite pulp, thermomechanical pulp, and the like. Further, the wood fibers may be any high-average fiber length wood pulp, low-average fiber length wood pulp, or mixtures of the same. One example of suitable high-average length wood pulp fibers includes softwood fibers such as, but not limited to, northern softwood, southern softwood, redwood, red cedar, hemlock, pine (e.g., southern pines), spruce (e.g., black spruce), combinations thereof, and the like. The present invention is particularly suitable to employ low-average length wood pulp fibers, such as hardwood fibers, in particular, but not limited to, eucalyptus, maple, birch, aspen, and the like. The fibers may be treated so as to allow optimization of processing and or absorbency properties, The term "treated" as used herein is understood to include any means of introducing the additive to the fiber and/or fibrous matrix, but not limited to, such as coating, spraying, printing, chemical modifications, wet-end additions applications to the fibers as well as blending untreated fibers with treated fibers.

Moreover, if desired, secondary fibers obtained from recycled materials may be used, such as fiber pulp from sources such as, for example, newsprint, reclaimed paperboard, and office waste, or recycled diapers, be it from factory scrap or be it post-consumer recycling.

The fibrous material may be delivered as fibrous board or in fibrous sheet form. Also bales of fibrous material may be delivered as may be opened prior to be used in the process according to the present invention. Further, "roughly graded material" may be employed, wherein fibers are present as clusters of several hundreds up to several thousand fibers in the roughly graded material. Articles as treated in an apparatus according to the present invention comprise such fibrous webs and typically further materials, typically web materials for stabilizing and/or containing the fibrous webs, whereby the web sections may be laminated between envelope webs, such as topsheets and or backsheet in the case of absorbent articles.

Conventional air-laying processes are often operated in an "off-line" modus, whereby jumbo reels of the fibrous structure are produced on machines with a large width. Such wide roll may be cut to a lower width and rewound for being transferred to a converter, where the fibrous materials are cut to the appropriate size for being introduced into articles, often absorbent articles, such as wipes, or pads like bed-pads, or the like.

Whilst such processes allow efficient production of the fibrous webs, rewinding and transport imply process complexity and cost. When avoiding this by producing airlaid webs in-line on the converting process, conventional air-laying processes provide continuous webs that imply potential for contamination when being cut to the right length, and further require cut and space operation to feed into the finished articles.

Optionally, and in particular for the use in absorbent articles, the fibers may be combined with particles, especially superabsorbent polymer (SAP) particles, be this by intermixing fibers and particles, or by positioning the particles on a formed layer of fibers, preferably an unbonded layer, and often covered by a further fibrous layer. Thus, within the present context, the term "fiber/particles" refers to a pure fibrous structure or to any of such a fiber particle combination.

In order to further explain the equipment of and the method for executing the present invention, reference is made to the figures, which however, should not be seen to limit the present invention. In particular may features as explained with reference to one figure also be employed in the context of other figures.

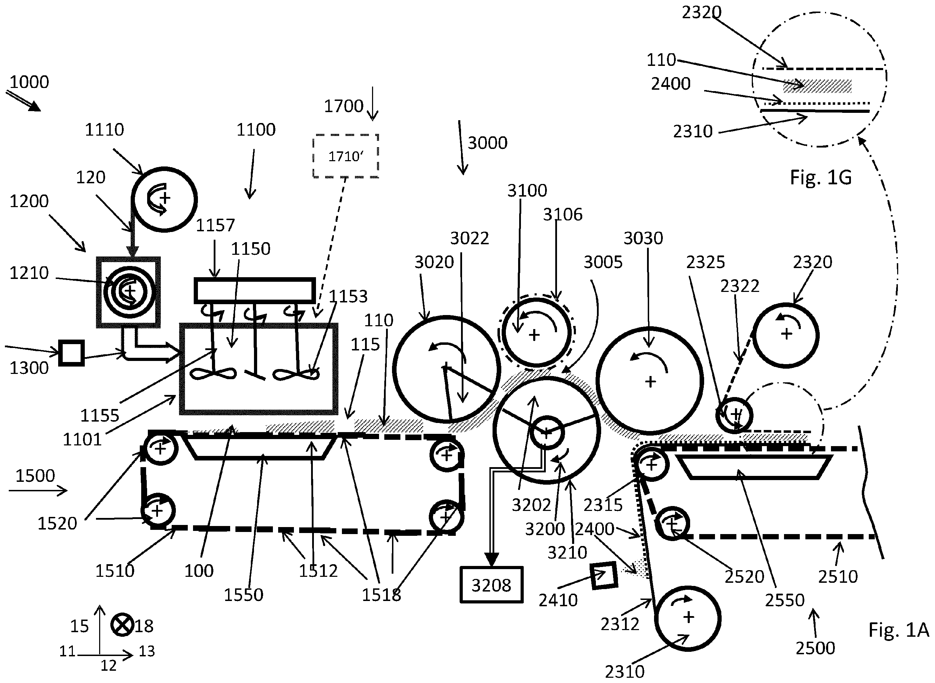

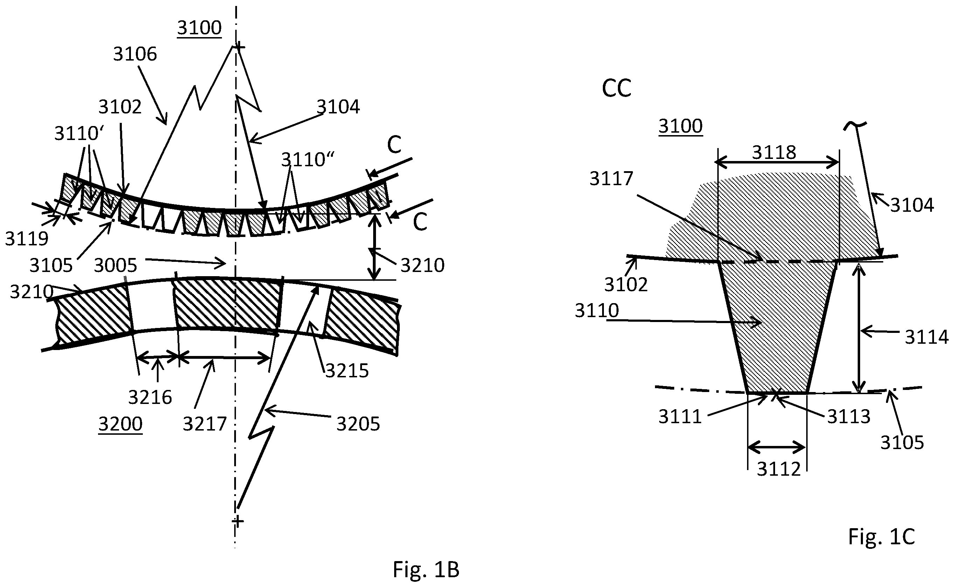

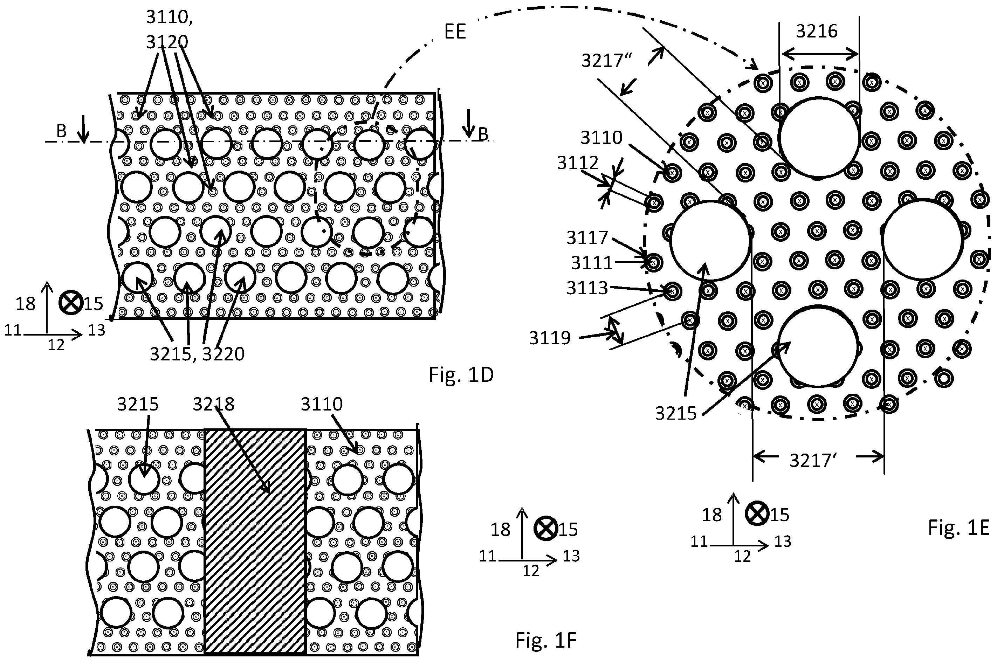

Fig. 1 depicts exemplarily without implying any limitation thereto a well-known equipment 1000 for forming a fibrous web, comprising a fiber supply 1110, e.g., an unwinder 1111 for delivering a fiber board 120 to a disintegration unit 1200, such as a hammer mill 1210. Alternatively, the fibers may be delivered from other fiber supply means (not shown) such as when being delivered in bale form or from a silo or bin. Disintegrated fibers are transferred, preferably pneumatically by means of air supply 1300, towards the forming box 1101 of a web forming unit 1100. As depicted, the web forming unit may comprise fiber homogenizer 1150, such as fans 1153 rotating by drive 1157 via vertical axes 1155. Optionally, and often preferably, particulate material, such as superabsorbent polymer particles, may be fed by a particle application unit 1700 from a particle supply 1710 to the web forming unit 1100, where these may be well intermixed with fibers, whilst these are cross-directionally 18 and machine-directionally 12 homogenized. The fibers, and optionally particles, are further deposited through the forming box outlet onto a collection system 1500, forming an essentially unbonded continuous web that may be separated and spaced apart to form a series of separated web sections. In an often preferred alternative, separated web sections are formed during the laydown process. This is further exemplified in Fig. 1, with a collection system 1500 depicted as a belt collector unit comprising a belt 1510 driven and guided by dive and guide rolls 1520 and comprising air permeable regions 1512 air impermeable regions 1518. The collecting is further supported by a suction system 1550. The foraminous belt and the fibrous web sections 110 deposited there on are moving in the machine direction 12, transferring the web from the upstream region 11 towards the downstream 13 region of the equipment. Such a preparation of separate web section is further described in the above referenced EP24188478, to which express reference is made for an exemplary formation of separated web sections.

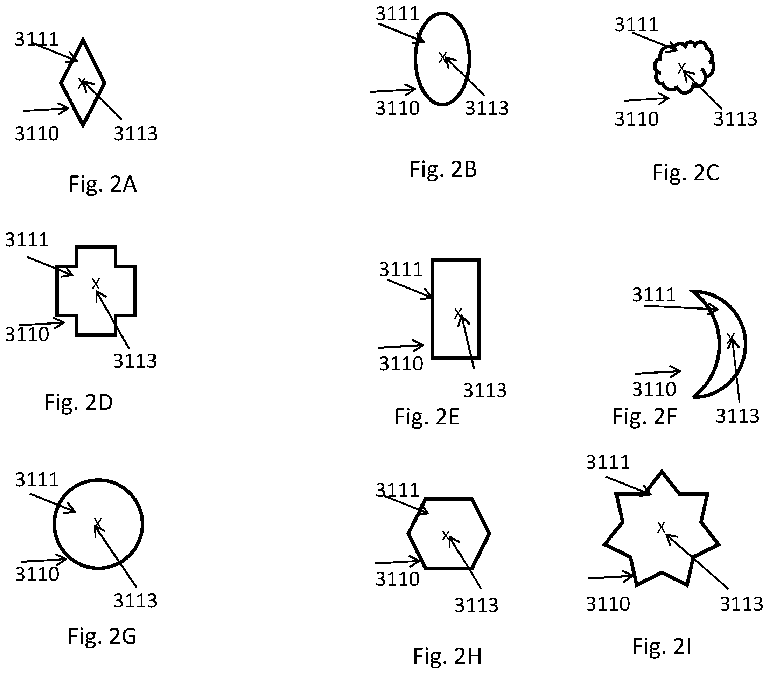

Whilst the creation of the series of web sections is not critical for the present invention, Fig. 1 A to G and 2 A to I further refer to the features according to the present invention, namely an embossing unit 3000 to be employed for embossing such fibrous web sections. To this end, the embossing unit 3000 comprises a vacuum anvil drum 3200 with a web support surface 3210, the surface comprising a multiplicity of apertures 3215 arranged in an aperture pattern 3220, preferably with equidistant (3217) apertures in a continuous pattern 3220 for the vacuum anvil drum. Preferably, all apertures have a circular shape but in any case, exhibit an equivalent diameter 3216 of less than about 10 mm, or less than about 5 mm, but more than about 1 mm, or more than about 2 mm. As the vacuum anvil drum is adapted to receive the series fiber/particle web sections 110 separated by fiber/particle free sections 115, the vacuum anvil drum preferably comprises aperture free-sections 3218 corresponding thereto. It is further contemplated that the aperture pattern 3220 may comprise apertures exhibiting non-circular shape (not shown) or that the aperture patterns comprise sub patterns with differently shaped, sized, or spaced apertures. The vacuum anvil drum 3200 further comprises a stationary vacuum section 3202, connected to a vacuum source 3208, such that the vacuum suction is restricted to the region where the web sections are positioned for the embossing. A counteracting embossing drum 3100 comprises an outer surface 3102, from which a multiplicity of raised embossing protrusions 3110 extend radially outwardly from a protrusion base 3117 of a size 3118, each protrusion exhibiting an outwardly oriented embossing surface 3111, together forming the geometrically enveloping cylinder surface 3105 circumscribing the raised embossing protrusions 3110, whereby the raised embossing protrusion surfaces 3111 forming an embossing protrusion surfaces pattern 3120.

Fig. 1C depicts exemplarily an enlarged cross-sectional view of a protrusion 3110, as may exhibit a truncated cone with a protrusion base 3117 as may exhibit a circular protrusion base diameter 3118 and an outwardly positioned protrusion surface 3111, circumscribed by the enveloping cylinder surface 3105 of the embossing drum. The embossing protrusions may exhibit a height 3114 of typically more than about 0.2 mm, or more than about 0.5 mm, but less than about 8 mm or less than about 5 mm. The shape of the protrusion surfaces 3111 is not particularly limited, see exemplary executions in Fig. 2A to 2I, all exhibiting a protrusion surface are as may be expressed by an equivalent diameter 3113 and a geometric center point 3113. Preferably, the equivalent diameter is more than about 0.15 mm, or more than about 0.5 mm, but less than about 20.0 mm or less than about 12.0 mm. In a preferred execution the protrusion surfaces exhibit a circular or elliptic shape. The distance 3119 of any geometric center point 3113 to the one of the any neighbouring raised embossing protrusion surface center point 3119 is less than about 5 mm, or less than about 3 mm, or less than about 1.5mm, but typically more than about 0.25 mm or more than about 0.5 mm. The shape of the embossing surfaces may be the same for all, although the embossing surface pattern 3120 may comprise two or more sub-regions (not shown) with different shapes of embossing surfaces. There may be a single embossing pattern 3120 for the total surface of the embossing drum 3102, although the embossing pattern 3112 may comprise two or more interlacing or adjacent embossing pattern sub-regions.

However, it is important that the apertures 3215 and the raised protrusions 3110 are arranged such that they are not in a facing position during operation. To this end, the apertures 3215 and the raised protrusion surfaces 3111 do not overlap when the flat projection of the aperture pattern 3220 and the embossing protrusions surfaces pattern 3120 are overlaid. Referring to Fig. 1D and the enlarged portion in Fig. 1E, both the aperture pattern 3220 of the larger apertures 3215, here shown as circles, and the embossing surface pattern 3120 of the smaller embossing protrusion surfaces 3111 are shown equidistantly and constant throughout the surfaces.

Referring to Fig. 1B, a cross-sectional view across the embossing gap 3005 between the embossing drum 3100 and vacuum anvil drum 3200 corresponding to line BB of Fig. 1D is shown. A first plurality of embossing protrusions 3110' are shown as shaded areas, because the cross-sectional line cuts through the embossing protrusion 3110', whilst protrusions of a second plurality of embossing protrusions 3110" are seen as contour of the protrusions of an adjacent protrusion pattern line. Thus, no protrusion 3110 will be in a facing position to an aperture 3215.

As neighbouring web sections 110 delivered to the embossing unit 3000 are separated by fiber and particle free regions 115, this is reflected by corresponding sections of the vacuum anvil drum by providing a flat surface region 3218 phased with the fiber and particle free regions 115. Referring to Fig. 1A for explaining the operating of the embossing unit 3000, the web sections 110 are fed to the gap 3005 between the vacuum anvil drum 3200 and the embossing drum 3100. This may be achieved by a first transfer drum 3020, preferably with a foraminous air permeable drum surface and a first stationary vacuum section 3022. First transfer drum and first stationary vacuum section 3022 are positioned so as to pick up the web sections 110 at or close to the downstream (13) end of the collecting belt 1510. Upon rotating, the web sections 110 reach the gap 3005 between the first transfer drum 3020 and the vacuum anvil drum 3200, whereby the vacuum sections 3022 and 3202, respectively, are sized and positioned so as to enable a smooth transfer of the web sections. The blocked sections 3218 of the vacuum anvil drum are also phased with the fiber and particle free regions 115 between the web sections 110.

Upon further rotation of the vacuum anvil drum 3200, the web sections 110 are fed into the gap 3005 between the vacuum transfer drum 3200 and the embossing drum 3100. The gap width 3004 between the surface of the embossing drum 3102, from which the protrusions 3110, exhibiting a protrusion height 3114, protrude, and the surface of the vacuum anvil drum 3210 depends on the thickness of the web, and may vary from about 1 mm to about 0.005 mm.

Because of the matched patterns of the embossing protrusions (3120) and the apertures (3220), respectively, it is a particular effect of the present invention that the fibers of web sections 110 are compressed and thereby bonded to each other, such as by hydrogen bonding, by the embossing protrusions 3110, but not lost through the apertures by the vacuum, which would be aggravated by the push of the embossing protrusions. The particular match of the aperture pattern 3220 and the protrusion pattern 3120 can be achieved by the above selection of
- of the distances between neighbouring embossing protrusions 3110;
- the size of the apertures 3216;
- the size 3112 of the protrusion surfaces 3211;
- and the distance 3119 between the center points 3113 of the protrusion surfaces 3111.

This allows to use short fibers, in particular also short length fibers of less than about 1.5 mm, or less than about 1 mm, such as eucalyptus fibers or other short fibers as described in the above, to be processes without excessive fiber losses to the vacuum system.

After compressing and bonding of the web sections 110, these may be transferred by a further transfer vacuum drum 3030 to further downstream (13) processing. In the exemplary presentation in Fig. 1A, the compressed web sections 110 are positioned onto a further web transport belt system 2500, comprising an endless foraminous belt 2510, vacuum suction units 2550 and guide and drive rolls 2520.

An exemplary processing may be performed on a laminating unit 2000 for enveloping the bonded web sections 110 between envelop webs. In the depicted execution, a first envelope web 2312, as may functions as a topsheet in the finished article, is provided from a first envelope supply 2310. As shown as an often preferred option, an first adhesive 2400 may be applied to a surface of the first envelope web 2312 by a first glue applicator 2410 during its transfer towards a gap between the further transfer drum 3030 and the further web transport belt system 2500. Further downstream, a second envelope web 2322, as may functions as a backsheet in the finished article, may be supplied from a second envelope web supply 2320 and optionally treated by a second glue applicator (not shown) during its transfer towards a combining roll 2325, which may also function as a final combining roll for connecting the envelope webs 2312, 2322 in the periphery around the compressed web sections 110. Final articles may then be separated by final knives (not shown), optionally folded and packed, e.g., by stacking units, into bags or boxes.

In order to not exhibit undue stress to the web sections, the operational speeds of the web transfer systems and the embossing rolls need to be synchronized both with regard to speed and position.

## Claims

1. An embossing unit (3000) for embossing fibrous web sections (110), said embossing unit (3000) comprising
- a vacuum anvil drum (3200) with a web support surface (3210), said web support surface (3210) comprising a multiplicity of apertures (3215) arranged in an aperture pattern (3220), and
- a counteracting embossing drum (3100) comprising on its outer surface (3120) a multiplicity of raised embossing protrusions (3110), each exhibiting an outwardly oriented embossing surface (3111) on the enveloping cylinder surface (3105) circumscribing said raised embossing protrusions (3110),
said raised embossing protrusion surfaces (3111) forming an embossing protrusions surface pattern (3120);
whereby
- said apertures (3215) exhibit an equivalent diameter of less than about 10 mm,
- said raised embossing protrusions (3110) exhibit an outwardly oriented embossing surface (3111) exhibiting a circular equivalent area of
∘ less than about 20 mm,
∘ and more than about 0.15 mm;
- said raised embossing protrusions surfaces exhibit a center point (3113') to neighbouring center point (3113") distance of less than about 5 mm;
- said apertures (3215) and said raised protrusions (3119) are arranged such that in a juxtaposition of a flat projection of said aperture pattern (3220) and said embossing protrusion surfaces pattern (3120) said apertures (3215) and said protrusion surfaces (3111) do not overlap.

2. An embossing unit (3000) for embossing fibrous web sections (110) according to claim 1, wherein said anvil (3200) and said embossing roll (3100) are adapted to be operated at synchronized speed and circular position.

3. An embossing unit (3000) for embossing fibrous web sections (110) according to claim 1 or 2, whereby said aperture pattern (3220) and said embossing protrusion surfaces pattern (3120) comprise separation regions (3218) free of apertures and embossing protrusions.

4. An embossing unit (3000) for embossing fibrous web sections (110) according to any of the preceding claims, wherein said raised protrusion surfaces (3111) exhibit a shape selected from the group consisting of
- circle, oval, polygon, star, rhomb, triangle, crescent,
- preferably a circle or oval.

5. A process for embossing a series of separated fibrous web sections (110), preferably liquid absorbent fibrous web sections,
said process comprising the steps of
- providing manufacturing units of
o a web pieces supply unit (1000), preferably a foraminous belt system (1500) for providing a series of essentially unbonded fibrous web sections (110), preferably air-laid web pieces,
▪ said web sections (110) comprising
▪ short fibers, preferably cellulosic fibers, preferably exhibiting an average fiber length of less than about 1.5 mm, more preferably of less than about 1.0 mm,
▪ and optionally particulate material, preferably superabsorbent material,
∘ an embossing unit (3000) for embossing said series of separated fibrous web sections,
▪ comprising
• a vacuum anvil drum (3200) with a web support surface (3210), said web support surface (3210) comprising a multiplicity / plurality of apertures (3215), arranged in an aperture pattern (3220),
• and
• a counteracting embossing drum (3100) comprising on its outer surface (3102) a multiplicity of raised embossing protrusions (3110) exhibiting an outwardly oriented embossing surface (3111) on the enveloping cylinder surface (3105) circumscribing said raised embossing protrusions (3110), said raised embossing protrusions (3110) forming an embossing protrusions pattern (3120);
∘ a first transfer unit (3020) for transferring said series of separated web sections (110) to said embossing unit (3000), preferably a vacuum transfer drum;
∘ a first envelope web supply (2310) for providing a continuous envelope web (2312), preferably a fluid pervious web and preferably a first envelope web glue applicator (2410) for applying glue (2400) to said first envelope web (2312);
∘ optionally a further envelope web supply (2320) for providing a continuous envelope web (2322), preferably a fluid impervious web and a further envelope web glue applicator for applying glue (2400) to said further envelope web (2322);
∘ a transfer and combining unit (3030, 2500) for receiving said embossed web sections (110) from said embossing unit (3000) and for combing these with said envelope web(s) (2312, 2322);
∘ a web removal unit, preferably a foraminous belt system (2500),
whereby said manufacturing units are arranged to define an upstream (11) to downstream (13) path
∘ from said web pieces supply,
∘ to said transfer unit;
∘ to said embossing unit
∘ to said further transfer and combining unit
∘ to said web removal unit;
- delivering said series of fibrous web sections (110) on said supply unit (1500) towards said first transfer unit/ drum;
- transferring said fibrous web sections (110) by said first transfer unit (3030) to said embossing unit (3000);
- embossing said fibrous web sections (110) by said embossing unit (3000) in regions of said protrusion pattern (3120) but not in regions of said aperture pattern (3220),
∘ thereby operating said embossing unit (3030) synchronized in speed and rotative position to said series of fibrous web sections (110);
- optionally applying glue (2400) to said first envelope web (2312), preferably in a pattern corresponding in length and position to said series of web sections
- transferring said embossed web sections (110) and said carrier web to said transfer and combing unit (3030, 2500);
- transferring said carrier web with said web pieces towards said removal unit and to further downstream processing units.

6. A process for embossing a series of separated fibrous web sections (110) according to claim 5, thereby operating said embossing unit (3000) so as to increase the internal strength of said web sections (110) by creating hydrogen bonding between fibers of said fibrous web sections (110).
